# EUROPEAN PATENT APPLICATION

(11) **EP 1 956 029 A1**
(43) Date of publication of application: **13.08.2008**
(21) Application number: 06822567.1
(22) Date of filing: 30.10.2006
(51) Int. Cl.: C07K 14/47, C07K 16/18, G01N 33/53

(54) **VASCULAR AGING-PREDICTING FACTOR AND UTILIZATION OF THE SAME**

(30) Priority: 29.11.2005 JP 2005344630
(71) Applicant: Nagasaki University, Nagasaki-Shi Nagasaki , 8528521 (JP)
(72) Inventor: KONDO, Takahito, Nagasaki-shi, Nagasaki 852- 8521 (JP); IHARA, Yoshihito, Nagasaki-shi, Nagasaki 852-8521 (JP); URATA, Yoshishige, Nagasaki-shi, Nagasaki 852-8521 (JP); GOTO, Shinji, Nagasaki-shi, Nagasaki 852-8521 (JP); KONO, Takaaki, Nagasaki-shi, Nagasaki 852-8521 (JP)
(74) Representative: Duckworth, Timothy John
(86) International application number: PCT/JP2006/321610
(87) International publication number: WO 2007/063664

(57) **Abstract**

A predicting factor for vascular aging and an examination method for an early-stage lesion resulting from vascular aging are provided. Specifically, a predicting factor for vascular aging comprising a human apolipoprotein B100 having a glutathionylated thiol group; a method of examining a lesion resulting from vascular aging contain measurement of a human apolipoprotein B100 having a glutathionylated thiol group in the blood sample; an antibody specifically recognizing an apolipoprotein B100 having a glutathionylated thiol group; a diagnostic agent or a diagnostic kit for an early-stage lesion resulting from vascular aging containing an antibody recognizing a human apolipoprotein B100 having a glutathionylated thiol group, are provided.

SEQUENCE LISTING

<110>  NAGASAKI UNIVERSITY

<120>  VASCULAR AGING-PREDICTING FACTOR AND UTILIZATION OF THE SAME

<130>  091000

<150>  JP2005-344630
<151>  2005-11-29

<160>  2

<170>  PatentIn version 3.2

<210>  1
<211>  4536
<212>  PRT
<213>  Homo sapiens

<400>  1

Glu Glu Glu Met Leu Glu Asn Val Ser Leu Val Cys Pro Lys Asp Ala
1               5                   10                  15

Thr Arg Phe Lys His Leu Arg Lys Tyr Thr Tyr Asn Tyr Glu Ala Glu
            20                  25                  30

Ser Ser Ser Gly Val Pro Gly Thr Ala Asp Ser Arg Ser Ala Thr Arg
        35                  40                  45

Ile Asn Cys Lys Val Glu Leu Glu Val Pro Gln Leu Cys Ser Phe Ile
    50                  55                  60

Leu Lys Thr Ser Gln Cys Thr Leu Lys Glu Val Tyr Gly Phe Asn Pro
65                  70                  75                  80

Glu Gly Lys Ala Leu Leu Lys Lys Thr Lys Asn Ser Glu Glu Phe Ala
                85                  90                  95

Ala Ala Met Ser Arg Tyr Glu Leu Lys Leu Ala Ile Pro Glu Gly Lys
            100                 105                 110

Gln Val Phe Leu Tyr Pro Glu Lys Asp Glu Pro Thr Tyr Ile Leu Asn
        115                 120                 125

Ile Lys Arg Gly Ile Ile Ser Ala Leu Leu Val Pro Pro Glu Thr Glu
    130                 135                 140

Glu Ala Lys Gln Val Leu Phe Leu Asp Thr Val Tyr Gly Asn Cys Ser
145                 150                 155                 160

Thr His Phe Thr Val Lys Thr Arg Lys Gly Asn Val Ala Thr Glu Ile
                165                 170                 175

Ser Thr Glu Arg Asp Leu Gly Gln Cys Asp Arg Phe Lys Pro Ile Arg
            180                 185                 190

Thr Gly Ile Ser Pro Leu Ala Leu Ile Lys Gly Met Thr Arg Pro Leu
        195                 200                 205

Ser Thr Leu Ile Ser Ser Ser Gln Ser Cys Gln Tyr Thr Leu Asp Ala
    210                 215                 220

Lys Arg Lys His Val Ala Glu Ala Ile Cys Lys Glu Gln His Leu Phe
225                 230                 235                 240

Leu Pro Phe Ser Tyr Asn Asn Lys Tyr Gly Met Val Ala Gln Val Thr
                245                 250                 255

Gln Thr Leu Lys Leu Glu Asp Thr Pro Lys Ile Asn Ser Arg Phe Phe
            260                 265                 270

Gly Glu Gly Thr Lys Lys Met Gly Leu Ala Phe Glu Ser Thr Lys Ser
        275                 280                 285

Thr Ser Pro Pro Lys Gln Ala Glu Ala Val Leu Lys Thr Leu Gln Glu
    290                 295                 300

Leu Lys Lys Leu Thr Ile Ser Glu Gln Asn Ile Gln Arg Ala Asn Leu
305                 310                 315                 320

Phe Asn Lys Leu Val Thr Glu Leu Arg Gly Leu Ser Asp Glu Ala Val
                325                 330                 335

Thr Ser Leu Leu Pro Gln Leu Ile Glu Val Ser Ser Pro Ile Thr Leu
            340                 345                 350

Gln Ala Leu Val Gln Cys Gly Gln Pro Gln Cys Ser Thr His Ile Leu
        355                 360                 365

Gln Trp Leu Lys Arg Val His Ala Asn Pro Leu Leu Ile Asp Val Val
    370                 375                 380

Thr Tyr Leu Val Ala Leu Ile Pro Glu Pro Ser Ala Gln Gln Leu Arg
385                 390                 395                 400

Glu Ile Phe Asn Met Ala Arg Asp Gln Arg Ser Arg Ala Thr Leu Tyr
                405                 410                 415

Ala Leu Ser His Ala Val Asn Asn Tyr His Lys Thr Asn Pro Thr Gly
            420                 425                 430

Thr Gln Glu Leu Leu Asp Ile Ala Asn Tyr Leu Met Glu Gln Ile Gln
        435                 440                 445

Asp Asp Cys Thr Gly Asp Glu Asp Tyr Thr Tyr Leu Ile Leu Arg Val
    450                 455                 460

Ile Gly Asn Met Gly Gln Thr Met Glu Gln Leu Thr Pro Glu Leu Lys
465                 470                 475                 480

Ser Ser Ile Leu Lys Cys Val Gln Ser Thr Lys Pro Ser Leu Met Ile
                485                 490                 495

Gln Lys Ala Ala Ile Gln Ala Leu Arg Lys Met Glu Pro Lys Asp Lys
            500                 505                 510

Asp Gln Glu Val Leu Leu Gln Thr Phe Leu Asp Asp Ala Ser Pro Gly
        515                 520                 525

Asp Lys Arg Leu Ala Ala Tyr Leu Met Leu Met Arg Ser Pro Ser Gln
    530                 535                 540

Ala Asp Ile Asn Lys Ile Val Gln Ile Leu Pro Trp Glu Gln Asn Glu
545                 550                 555                 560

Gln Val Lys Asn Phe Val Ala Ser His Ile Ala Asn Ile Leu Asn Ser
                565                 570                 575

Glu Glu Leu Asp Ile Gln Asp Leu Lys Lys Leu Val Lys Glu Ala Leu
            580                 585                 590

Lys Glu Ser Gln Leu Pro Thr Val Met Asp Phe Arg Lys Phe Ser Arg
        595                 600                 605

Asn Tyr Gln Leu Tyr Lys Ser Val Ser Leu Pro Ser Leu Asp Pro Ala
    610                 615                 620

Ser Ala Lys Ile Glu Gly Asn Leu Ile Phe Asp Pro Asn Asn Tyr Leu
625                 630                 635                 640

Pro Lys Glu Ser Met Leu Lys Thr Thr Leu Thr Ala Phe Gly Phe Ala
                645                 650                 655

Ser Ala Asp Leu Ile Glu Ile Gly Leu Glu Gly Lys Gly Phe Glu Pro
            660                 665                 670

Thr Leu Glu Ala Leu Phe Gly Lys Gln Gly Phe Phe Pro Asp Ser Val
        675                 680                 685

Asn Lys Ala Leu Tyr Trp Val Asn Gly Gln Val Pro Asp Gly Val Ser
    690                 695                 700

Lys Val Leu Val Asp His Phe Gly Tyr Thr Lys Asp Asp Lys His Glu
705                 710                 715                 720

Gln Asp Met Val Asn Gly Ile Met Leu Ser Val Glu Lys Leu Ile Lys
                725                 730                 735

Asp Leu Lys Ser Lys Glu Val Pro Glu Ala Arg Ala Tyr Leu Arg Ile
            740                 745                 750

Leu Gly Glu Glu Leu Gly Phe Ala Ser Leu His Asp Leu Gln Leu Leu
        755                 760                 765

Gly Lys Leu Leu Leu Met Gly Ala Arg Thr Leu Gln Gly Ile Pro Gln
    770                 775                 780

Met Ile Gly Glu Val Ile Arg Lys Gly Ser Lys Asn Asp Phe Phe Leu
785                 790                 795                 800

His Tyr Ile Phe Met Glu Asn Ala Phe Glu Leu Pro Thr Gly Ala Gly
                805                 810                 815

Leu Gln Leu Gln Ile Ser Ser Ser Gly Val Ile Ala Pro Gly Ala Lys
            820                 825                 830

Ala Gly Val Lys Leu Glu Val Ala Asn Met Gln Ala Glu Leu Val Ala
        835                 840                 845

Lys Pro Ser Val Ser Val Glu Phe Val Thr Asn Met Gly Ile Ile Ile
    850                 855                 860

Pro Asp Phe Ala Arg Ser Gly Val Gln Met Asn Thr Asn Phe Phe His
865                 870                 875                 880

Glu Ser Gly Leu Glu Ala His Val Ala Leu Lys Ala Gly Lys Leu Lys
                885                 890                 895

Phe Ile Ile Pro Ser Pro Lys Arg Pro Val Lys Leu Leu Ser Gly Gly
            900                 905                 910

Asn Thr Leu His Leu Val Ser Thr Thr Lys Thr Glu Val Ile Pro Pro
        915                 920                 925

Leu Ile Glu Asn Arg Gln Ser Trp Ser Val Cys Lys Gln Val Phe Pro
    930                 935                 940

Gly Leu Asn Tyr Cys Thr Ser Gly Ala Tyr Ser Asn Ala Ser Ser Thr
945                 950                 955                 960

Asp Ser Ala Ser Tyr Tyr Pro Leu Thr Gly Asp Thr Arg Leu Glu Leu
                965                 970                 975

Glu Leu Arg Pro Thr Gly Glu Ile Glu Gln Tyr Ser Val Ser Ala Thr
            980                 985                 990

Tyr Glu Leu Gln Arg Glu Asp Arg Ala Leu Val Asp Thr Leu Lys Phe
        995                 1000                1005

Val Thr Gln Ala Glu Gly Ala Lys Gln Thr Glu Ala Thr Met Thr
    1010                1015                1020

Phe Lys Tyr Asn Arg Gln Ser Met Thr Leu Ser Ser Glu Val Gln
    1025                1030                1035

Ile Pro Asp Phe Asp Val Asp Leu Gly Thr Ile Leu Arg Val Asn
    1040                1045                1050

Asp Glu Ser Thr Glu Gly Lys Thr Ser Tyr Arg Leu Thr Leu Asp
    1055                1060                1065

Ile Gln Asn Lys Lys Ile Thr Glu Val Ala Leu Met Gly His Leu
    1070                1075                1080

Ser Cys Asp Thr Lys Glu Glu Arg Lys Ile Lys Gly Val Ile Ser

1085                1090                1095

Ile Pro Arg Leu Gln Ala Glu Ala Arg Ser Glu Ile Leu Ala His
    1100                1105                1110

Trp Ser Pro Ala Lys Leu Leu Leu Gln Met Asp Ser Ser Ala Thr
    1115                1120                1125

Ala Tyr Gly Ser Thr Val Ser Lys Arg Val Ala Trp His Tyr Asp
    1130                1135                1140

Glu Glu Lys Ile Glu Phe Glu Trp Asn Thr Gly Thr Asn Val Asp
    1145                1150                1155

Thr Lys Lys Met Thr Ser Asn Phe Pro Val Asp Leu Ser Asp Tyr
    1160                1165                1170

Pro Lys Ser Leu His Met Tyr Ala Asn Arg Leu Leu Asp His Arg
    1175                1180                1185

Val Pro Glu Thr Asp Met Thr Phe Arg His Val Gly Ser Lys Leu
    1190                1195                1200

Ile Val Ala Met Ser Ser Trp Leu Gln Lys Ala Ser Gly Ser Leu
    1205                1210                1215

Pro Tyr Thr Gln Thr Leu Gln Asp His Leu Asn Ser Leu Lys Glu
    1220                1225                1230

Phe Asn Leu Gln Asn Met Gly Leu Pro Asp Phe His Ile Pro Glu
    1235                1240                1245

Asn Leu Phe Leu Lys Ser Asp Gly Arg Val Lys Tyr Thr Leu Asn
    1250                1255                1260

Lys Asn Ser Leu Lys Ile Glu Ile Pro Leu Pro Phe Gly Gly Lys
    1265                1270                1275

Ser Ser Arg Asp Leu Lys Met Leu Glu Thr Val Arg Thr Pro Ala
    1280                1285                1290

Leu His Phe Lys Ser Val Gly Phe His Leu Pro Ser Arg Glu Phe
    1295                1300                1305

Gln Val Pro Thr Phe Thr Ile Pro Lys Leu Tyr Gln Leu Gln Val
    1310                1315                1320

Pro Leu Leu Gly Val Leu Asp Leu Ser Thr Asn Val Tyr Ser Asn
    1325                1330                1335

Leu Tyr Asn Trp Ser Ala Ser Tyr Ser Gly Gly Asn Thr Ser Thr
    1340                1345                1350

Asp His Phe Ser Leu Arg Ala Arg Tyr His Met Lys Ala Asp Ser
    1355                1360                1365

Val Val Asp Leu Leu Ser Tyr Asn Val Gln Gly Ser Gly Glu Thr
    1370                1375                1380

Thr Tyr Asp His Lys Asn Thr Phe Thr Leu Ser Cys Asp Gly Ser
    1385                1390                1395

Leu Arg His Lys Phe Leu Asp Ser Asn Ile Lys Phe Ser His Val
    1400                1405                1410

Glu Lys Leu Gly Asn Asn Pro Val Ser Lys Gly Leu Leu Ile Phe
    1415                1420                1425

Asp Ala Ser Ser Ser Trp Gly Pro Gln Met Ser Ala Ser Val His
    1430                1435                1440

Leu Asp Ser Lys Lys Lys Gln His Leu Phe Val Lys Glu Val Lys
    1445                1450                1455

Ile Asp Gly Gln Phe Arg Val Ser Ser Phe Tyr Ala Lys Gly Thr
    1460                1465                1470

Tyr Gly Leu Ser Cys Gln Arg Asp Pro Asn Thr Gly Arg Leu Asn
    1475                1480                1485

Gly Glu Ser Asn Leu Arg Phe Asn Ser Ser Tyr Leu Gln Gly Thr
    1490                1495                1500

Asn Gln Ile Thr Gly Arg Tyr Glu Asp Gly Thr Leu Ser Leu Thr
    1505                1510                1515

Ser Thr Ser Asp Leu Gln Ser Gly Ile Ile Lys Asn Thr Ala Ser
    1520                1525                1530

Leu Lys Tyr Glu Asn Tyr Glu Leu Thr Leu Lys Ser Asp Thr Asn
    1535                1540                1545

Gly Lys Tyr Lys Asn Phe Ala Thr Ser Asn Lys Met Asp Met Thr
    1550                1555                1560

Phe Ser Lys Gln Asn Ala Leu Leu Arg Ser Glu Tyr Gln Ala Asp
    1565                1570                1575

Tyr Glu Ser Leu Arg Phe Phe Ser Leu Leu Ser Gly Ser Leu Asn
    1580                1585                1590

Ser His Gly Leu Glu Leu Asn Ala Asp Ile Leu Gly Thr Asp Lys
    1595                1600                1605

Ile Asn Ser Gly Ala His Lys Ala Thr Leu Arg Ile Gly Gln Asp
    1610                1615                1620

Gly Ile Ser Thr Ser Ala Thr Thr Asn Leu Lys Cys Ser Leu Leu
    1625                1630                1635

Val Leu Glu Asn Glu Leu Asn Ala Glu Leu Gly Leu Ser Gly Ala
    1640                1645                1650

Ser Met Lys Leu Thr Thr Asn Gly Arg Phe Arg Glu His Asn Ala
    1655                1660                1665

Lys Phe Ser Leu Asp Gly Lys Ala Ala Leu Thr Glu Leu Ser Leu
    1670                1675                1680

Gly Ser Ala Tyr Gln Ala Met Ile Leu Gly Val Asp Ser Lys Asn
    1685                1690                1695

Ile Phe Asn Phe Lys Val Ser Gln Glu Gly Leu Lys Leu Ser Asn
    1700                1705                1710

Asp Met Met Gly Ser Tyr Ala Glu Met Lys Phe Asp His Thr Asn
    1715                1720                1725

Ser Leu Asn Ile Ala Gly Leu Ser Leu Asp Phe Ser Ser Lys Leu
    1730                1735                1740

Asp Asn Ile Tyr Ser Ser Asp Lys Phe Tyr Lys Gln Thr Val Asn
    1745                1750                1755

Leu Gln Leu Gln Pro Tyr Ser Leu Val Thr Thr Leu Asn Ser Asp
    1760                1765                1770

Leu Lys Tyr Asn Ala Leu Asp Leu Thr Asn Asn Gly Lys Leu Arg
    1775                1780                1785

Leu Glu Pro Leu Lys Leu His Val Ala Gly Asn Leu Lys Gly Ala
    1790                1795                1800

Tyr Gln Asn Asn Glu Ile Lys His Ile Tyr Ala Ile Ser Ser Ala
    1805                1810                1815

Ala Leu Ser Ala Ser Tyr Lys Ala Asp Thr Val Ala Lys Val Gln
    1820                1825                1830

Gly Val Glu Phe Ser His Arg Leu Asn Thr Asp Ile Ala Gly Leu
    1835                1840                1845

Ala Ser Ala Ile Asp Met Ser Thr Asn Tyr Asn Ser Asp Ser Leu
    1850                1855                1860

His Phe Ser Asn Val Phe Arg Ser Val Met Ala Pro Phe Thr Met
    1865                1870                1875

Thr Ile Asp Ala His Thr Asn Gly Asn Gly Lys Leu Ala Leu Trp
    1880                1885                1890

Gly Glu His Thr Gly Gln Leu Tyr Ser Lys Phe Leu Leu Lys Ala
    1895                1900                1905

Glu Pro Leu Ala Phe Thr Phe Ser His Asp Tyr Lys Gly Ser Thr
    1910                1915                1920

Ser His His Leu Val Ser Arg Lys Ser Ile Ser Ala Ala Leu Glu
    1925                1930                1935

His Lys Val Ser Ala Leu Leu Thr Pro Ala Glu Gln Thr Gly Thr
    1940                1945                1950

Trp Lys Leu Lys Thr Gln Phe Asn Asn Asn Glu Tyr Ser Gln Asp
    1955                1960                1965

Leu Asp Ala Tyr Asn Thr Lys Asp Lys Ile Gly Val Glu Leu Thr
    1970                1975                1980

Gly Arg Thr Leu Ala Asp Leu Thr Leu Leu Asp Ser Pro Ile Lys
    1985                1990                1995

Val Pro Leu Leu Leu Ser Glu Pro Ile Asn Ile Ile Asp Ala Leu
    2000                2005                2010

Glu Met Arg Asp Ala Val Glu Lys Pro Gln Glu Phe Thr Ile Val
    2015                2020                2025

Ala Phe Val Lys Tyr Asp Lys Asn Gln Asp Val His Ser Ile Asn
    2030                2035                2040

Leu Pro Phe Phe Glu Thr Leu Gln Glu Tyr Phe Glu Arg Asn Arg
    2045                2050                2055

Gln Thr Ile Ile Val Val Val Glu Asn Val Gln Arg Asn Leu Lys
    2060                2065                2070

His Ile Asn Ile Asp Gln Phe Val Arg Lys Tyr Arg Ala Ala Leu
    2075                2080                2085

Gly Lys Leu Pro Gln Gln Ala Asn Asp Tyr Leu Asn Ser Phe Asn
    2090                2095                2100

Trp Glu Arg Gln Val Ser His Ala Lys Glu Lys Leu Thr Ala Leu
    2105                2110                2115

Thr Lys Lys Tyr Arg Ile Thr Glu Asn Asp Ile Gln Ile Ala Leu
    2120                2125                2130

Asp Asp Ala Lys Ile Asn Phe Asn Glu Lys Leu Ser Gln Leu Gln
    2135                2140                2145

Thr Tyr Met Ile Gln Phe Asp Gln Tyr Ile Lys Asp Ser Tyr Asp
    2150                2155                2160

Leu His Asp Leu Lys Ile Ala Ile Ala Asn Ile Ile Asp Glu Ile
    2165                2170                2175

Ile Glu Lys Leu Lys Ser Leu Asp Glu His Tyr His Ile Arg Val
    2180                2185                2190

Asn Leu Val Lys Thr Ile His Asp Leu His Leu Phe Ile Glu Asn
    2195                2200                2205

Ile Asp Phe Asn Lys Ser Gly Ser Ser Thr Ala Ser Trp Ile Gln
    2210                2215                2220

Asn Val Asp Thr Lys Tyr Gln Ile Arg Ile Gln Ile Gln Glu Lys

2225                    2230                    2235

Leu Gln Gln Leu Lys Arg His Ile Gln Asn Ile Asp Ile Gln His
    2240                    2245                    2250

Leu Ala Gly Lys Leu Lys Gln His Ile Glu Ala Ile Asp Val Arg
    2255                    2260                    2265

Val Leu Leu Asp Gln Leu Gly Thr Thr Ile Ser Phe Glu Arg Ile
    2270                    2275                    2280

Asn Asp Val Leu Glu His Val Lys His Phe Val Ile Asn Leu Ile
    2285                    2290                    2295

Gly Asp Phe Glu Val Ala Glu Lys Ile Asn Ala Phe Arg Ala Lys
    2300                    2305                    2310

Val His Glu Leu Ile Glu Arg Tyr Glu Val Asp Gln Gln Ile Gln
    2315                    2320                    2325

Val Leu Met Asp Lys Leu Val Glu Leu Thr His Gln Tyr Lys Leu
    2330                    2335                    2340

Lys Glu Thr Ile Gln Lys Leu Ser Asn Val Leu Gln Gln Val Lys
    2345                    2350                    2355

Ile Lys Asp Tyr Phe Glu Lys Leu Val Gly Phe Ile Asp Asp Ala
    2360                    2365                    2370

Val Lys Lys Leu Asn Glu Leu Ser Phe Lys Thr Phe Ile Glu Asp
    2375                    2380                    2385

Val Asn Lys Phe Leu Asp Met Leu Ile Lys Lys Leu Lys Ser Phe
    2390                    2395                    2400

Asp Tyr His Gln Phe Val Asp Glu Thr Asn Asp Lys Ile Arg Glu
    2405                    2410                    2415

Val Thr Gln Arg Leu Asn Gly Glu Ile Gln Ala Leu Glu Leu Pro
    2420                    2425                    2430

Gln Lys Ala Glu Ala Leu Lys Leu Phe Leu Glu Glu Thr Lys Ala
    2435                    2440                    2445

Thr Val Ala Val Tyr Leu Glu Ser Leu Gln Asp Thr Lys Ile Thr
    2450                    2455                    2460

Leu Ile Ile Asn Trp Leu Gln Glu Ala Leu Ser Ser Ala Ser Leu
    2465                    2470                    2475

Ala His Met Lys Ala Lys Phe Arg Glu Thr Leu Glu Asp Thr Arg
    2480                    2485                    2490

Asp Arg Met Tyr Gln Met Asp Ile Gln Gln Glu Leu Gln Arg Tyr
    2495                    2500                    2505

Leu Ser Leu Val Gly Gln Val Tyr Ser Thr Leu Val Thr Tyr Ile
    2510                    2515                    2520

Ser Asp Trp Trp Thr Leu Ala Ala Lys Asn Leu Thr Asp Phe Ala
    2525                    2530                    2535

Glu Gln Tyr Ser Ile Gln Asp Trp Ala Lys Arg Met Lys Ala Leu
    2540                    2545                    2550

Val Glu Gln Gly Phe Thr Val Pro Glu Ile Lys Thr Ile Leu Gly
    2555                    2560                    2565

Thr Met Pro Ala Phe Glu Val Ser Leu Gln Ala Leu Gln Lys Ala
    2570                    2575                    2580

Thr Phe Gln Thr Pro Asp Phe Ile Val Pro Leu Thr Asp Leu Arg
    2585                    2590                    2595

Ile Pro Ser Val Gln Ile Asn Phe Lys Asp Leu Lys Asn Ile Lys
    2600                    2605                    2610

Ile Pro Ser Arg Phe Ser Thr Pro Glu Phe Thr Ile Leu Asn Thr
    2615                2620                2625

Phe His Ile Pro Ser Phe Thr Ile Asp Phe Val Glu Met Lys Val
    2630                2635                2640

Lys Ile Ile Arg Thr Ile Asp Gln Met Gln Asn Ser Glu Leu Gln
    2645                2650                2655

Trp Pro Val Pro Asp Ile Tyr Leu Arg Asp Leu Lys Val Glu Asp
    2660                2665                2670

Ile Pro Leu Ala Arg Ile Thr Leu Pro Asp Phe Arg Leu Pro Glu
    2675                2680                2685

Ile Ala Ile Pro Glu Phe Ile Ile Pro Thr Leu Asn Leu Asn Asp
    2690                2695                2700

Phe Gln Val Pro Asp Leu His Ile Pro Glu Phe Gln Leu Pro His
    2705                2710                2715

Ile Ser His Thr Ile Glu Val Pro Thr Phe Gly Lys Leu Tyr Ser
    2720                2725                2730

Ile Leu Lys Ile Gln Ser Pro Leu Phe Thr Leu Asp Ala Asn Ala
    2735                2740                2745

Asp Ile Gly Asn Gly Thr Thr Ser Ala Asn Glu Ala Gly Ile Ala
    2750                2755                2760

Ala Ser Ile Thr Ala Lys Gly Glu Ser Lys Leu Glu Val Leu Asn
    2765                2770                2775

Phe Asp Phe Gln Ala Asn Ala Gln Leu Ser Asn Pro Lys Ile Asn
    2780                2785                2790

Pro Leu Ala Leu Lys Glu Ser Val Lys Phe Ser Ser Lys Tyr Leu
    2795                2800                2805

Arg Thr Glu His Gly Ser Glu Met Leu Phe Phe Gly Asn Ala Ile
    2810                2815                2820

Glu Gly Lys Ser Asn Thr Val Ala Ser Leu His Thr Glu Lys Asn
    2825                2830                2835

Thr Leu Glu Leu Ser Asn Gly Val Ile Val Lys Ile Asn Asn Gln
    2840                2845                2850

Leu Thr Leu Asp Ser Asn Thr Lys Tyr Phe His Lys Leu Asn Ile
    2855                2860                2865

Pro Lys Leu Asp Phe Ser Ser Gln Ala Asp Leu Arg Asn Glu Ile
    2870                2875                2880

Lys Thr Leu Leu Lys Ala Gly His Ile Ala Trp Thr Ser Ser Gly
    2885                2890                2895

Lys Gly Ser Trp Lys Trp Ala Cys Pro Arg Phe Ser Asp Glu Gly
    2900                2905                2910

Thr His Glu Ser Gln Ile Ser Phe Thr Ile Glu Gly Pro Leu Thr
    2915                2920                2925

Ser Phe Gly Leu Ser Asn Lys Ile Asn Ser Lys His Leu Arg Val
    2930                2935                2940

Asn Gln Asn Leu Val Tyr Glu Ser Gly Ser Leu Asn Phe Ser Lys
    2945                2950                2955

Leu Glu Ile Gln Ser Gln Val Asp Ser Gln His Val Gly His Ser
    2960                2965                2970

Val Leu Thr Ala Lys Gly Met Ala Leu Phe Gly Glu Gly Lys Ala
    2975                2980                2985

Glu Phe Thr Gly Arg His Asp Ala His Leu Asn Gly Lys Val Ile
    2990                2995                3000

Gly Thr Leu Lys Asn Ser Leu Phe Phe Ser Ala Gln Pro Phe Glu
    3005                3010                3015

Ile Thr Ala Ser Thr Asn Asn Glu Gly Asn Leu Lys Val Arg Phe
    3020                3025                3030

Pro Leu Arg Leu Thr Gly Lys Ile Asp Phe Leu Asn Asn Tyr Ala
    3035                3040                3045

Leu Phe Leu Ser Pro Ser Ala Gln Gln Ala Ser Trp Gln Val Ser
    3050                3055                3060

Ala Arg Phe Asn Gln Tyr Lys Tyr Asn Gln Asn Phe Ser Ala Gly
    3065                3070                3075

Asn Asn Glu Asn Ile Met Glu Ala His Val Gly Ile Asn Gly Glu
    3080                3085                3090

Ala Asn Leu Asp Phe Leu Asn Ile Pro Leu Thr Ile Pro Glu Met
    3095                3100                3105

Arg Leu Pro Tyr Thr Ile Ile Thr Thr Pro Pro Leu Lys Asp Phe
    3110                3115                3120

Ser Leu Trp Glu Lys Thr Gly Leu Lys Glu Phe Leu Lys Thr Thr
    3125                3130                3135

Lys Gln Ser Phe Asp Leu Ser Val Lys Ala Gln Tyr Lys Lys Asn
    3140                3145                3150

Lys His Arg His Ser Ile Thr Asn Pro Leu Ala Val Leu Cys Glu
    3155                3160                3165

Phe Ile Ser Gln Ser Ile Lys Ser Phe Asp Arg His Phe Glu Lys
    3170                3175                3180

Asn Arg Asn Asn Ala Leu Asp Phe Val Thr Lys Ser Tyr Asn Glu
    3185                3190                3195

Thr Lys Ile Lys Phe Asp Lys Tyr Lys Ala Glu Lys Ser His Asp
    3200                3205                3210

Glu Leu Pro Arg Thr Phe Gln Ile Pro Gly Tyr Thr Val Pro Val
    3215                3220                3225

Val Asn Val Glu Val Ser Pro Phe Thr Ile Glu Met Ser Ala Phe
    3230                3235                3240

Gly Tyr Val Phe Pro Lys Ala Val Ser Met Pro Ser Phe Ser Ile
    3245                3250                3255

Leu Gly Ser Asp Val Arg Val Pro Ser Tyr Thr Leu Ile Leu Pro
    3260                3265                3270

Ser Leu Glu Leu Pro Val Leu His Val Pro Arg Asn Leu Lys Leu
    3275                3280                3285

Ser Leu Pro His Phe Lys Glu Leu Cys Thr Ile Ser His Ile Phe
    3290                3295                3300

Ile Pro Ala Met Gly Asn Ile Thr Tyr Asp Phe Ser Phe Lys Ser
    3305                3310                3315

Ser Val Ile Thr Leu Asn Thr Asn Ala Glu Leu Phe Asn Gln Ser
    3320                3325                3330

Asp Ile Val Ala His Leu Leu Ser Ser Ser Ser Ser Val Ile Asp
    3335                3340                3345

Ala Leu Gln Tyr Lys Leu Glu Gly Thr Thr Arg Leu Thr Arg Lys
    3350                3355                3360

Arg Gly Leu Lys Leu Ala Thr Ala Leu Ser Leu Ser Asn Lys Phe

3365                    3370                    3375

Val Glu Gly Ser His Asn Ser Thr Val Ser Leu Thr Thr Lys Asn
    3380                    3385                    3390

Met Glu Val Ser Val Ala Lys Thr Thr Lys Ala Glu Ile Pro Ile
    3395                    3400                    3405

Leu Arg Met Asn Phe Lys Gln Glu Leu Asn Gly Asn Thr Lys Ser
    3410                    3415                    3420

Lys Pro Thr Val Ser Ser Ser Met Glu Phe Lys Tyr Asp Phe Asn
    3425                    3430                    3435

Ser Ser Met Leu Tyr Ser Thr Ala Lys Gly Ala Val Asp His Lys
    3440                    3445                    3450

Leu Ser Leu Glu Ser Leu Thr Ser Tyr Phe Ser Ile Glu Ser Ser
    3455                    3460                    3465

Thr Lys Gly Asp Val Lys Gly Ser Val Leu Ser Arg Glu Tyr Ser
    3470                    3475                    3480

Gly Thr Ile Ala Ser Glu Ala Asn Thr Tyr Leu Asn Ser Lys Ser
    3485                    3490                    3495

Thr Arg Ser Ser Val Lys Leu Gln Gly Thr Ser Lys Ile Asp Asp
    3500                    3505                    3510

Ile Trp Asn Leu Glu Val Lys Glu Asn Phe Ala Gly Glu Ala Thr
    3515                    3520                    3525

Leu Gln Arg Ile Tyr Ser Leu Trp Glu His Ser Thr Lys Asn His
    3530                    3535                    3540

Leu Gln Leu Glu Gly Leu Phe Phe Thr Asn Gly Glu His Thr Ser
    3545                    3550                    3555

Lys Ala Thr Leu Glu Leu Ser Pro Trp Gln Met Ser Ala Leu Val
    3560                    3565                    3570

Gln Val His Ala Ser Gln Pro Ser Ser Phe His Asp Phe Pro Asp
    3575                    3580                    3585

Leu Gly Gln Glu Val Ala Leu Asn Ala Asn Thr Lys Asn Gln Lys
    3590                    3595                    3600

Ile Arg Trp Lys Asn Glu Val Arg Ile His Ser Gly Ser Phe Gln
    3605                    3610                    3615

Ser Gln Val Glu Leu Ser Asn Asp Gln Glu Lys Ala His Leu Asp
    3620                    3625                    3630

Ile Ala Gly Ser Leu Glu Gly His Leu Arg Phe Leu Lys Asn Ile
    3635                    3640                    3645

Ile Leu Pro Val Tyr Asp Lys Ser Leu Trp Asp Phe Leu Lys Leu
    3650                    3655                    3660

Asp Val Thr Thr Ser Ile Gly Arg Arg Gln His Leu Arg Val Ser
    3665                    3670                    3675

Thr Ala Phe Val Tyr Thr Lys Asn Pro Asn Gly Tyr Ser Phe Ser
    3680                    3685                    3690

Ile Pro Val Lys Val Leu Ala Asp Lys Phe Ile Thr Pro Gly Leu
    3695                    3700                    3705

Lys Leu Asn Asp Leu Asn Ser Val Leu Val Met Pro Thr Phe His
    3710                    3715                    3720

Val Pro Phe Thr Asp Leu Gln Val Pro Ser Cys Lys Leu Asp Phe
    3725                    3730                    3735

Arg Glu Ile Gln Ile Tyr Lys Lys Leu Arg Thr Ser Ser Phe Ala

3740                    3745                    3750

Leu Asn Leu Pro Thr Leu Pro Glu Val Lys Phe Pro Glu Val Asp
    3755                    3760                    3765

Val Leu Thr Lys Tyr Ser Gln Pro Glu Asp Ser Leu Ile Pro Phe
    3770                    3775                    3780

Phe Glu Ile Thr Val Pro Glu Ser Gln Leu Thr Val Ser Gln Phe
    3785                    3790                    3795

Thr Leu Pro Lys Ser Val Ser Asp Gly Ile Ala Ala Leu Asp Leu
    3800                    3805                    3810

Asn Ala Val Ala Asn Lys Ile Ala Asp Phe Glu Leu Pro Thr Ile
    3815                    3820                    3825

Ile Val Pro Glu Gln Thr Ile Glu Ile Pro Ser Ile Lys Phe Ser
    3830                    3835                    3840

Val Pro Ala Gly Ile Val Ile Pro Ser Phe Gln Ala Leu Thr Ala
    3845                    3850                    3855

Arg Phe Glu Val Asp Ser Pro Val Tyr Asn Ala Thr Trp Ser Ala
    3860                    3865                    3870

Ser Leu Lys Asn Lys Ala Asp Tyr Val Glu Thr Val Leu Asp Ser
    3875                    3880                    3885

Thr Cys Ser Ser Thr Val Gln Phe Leu Glu Tyr Glu Leu Asn Val
    3890                    3895                    3900

Leu Gly Thr His Lys Ile Glu Asp Gly Thr Leu Ala Ser Lys Thr
    3905                    3910                    3915

Lys Gly Thr Leu Ala His Arg Asp Phe Ser Ala Glu Tyr Glu Glu
    3920                    3925                    3930

Asp Gly Lys Phe Glu Gly Leu Gln Glu Trp Glu Gly Lys Ala His
    3935                    3940                    3945

Leu Asn Ile Lys Ser Pro Ala Phe Thr Asp Leu His Leu Arg Tyr
    3950                    3955                    3960

Gln Lys Asp Lys Lys Gly Ile Ser Thr Ser Ala Ala Ser Pro Ala
    3965                    3970                    3975

Val Gly Thr Val Gly Met Asp Met Asp Glu Asp Asp Asp Phe Ser
    3980                    3985                    3990

Lys Trp Asn Phe Tyr Tyr Ser Pro Gln Ser Ser Pro Asp Lys Lys
    3995                    4000                    4005

Leu Thr Ile Phe Lys Thr Glu Leu Arg Val Arg Glu Ser Asp Glu
    4010                    4015                    4020

Glu Thr Gln Ile Lys Val Asn Trp Glu Glu Glu Ala Ala Ser Gly
    4025                    4030                    4035

Leu Leu Thr Ser Leu Lys Asp Asn Val Pro Lys Ala Thr Gly Val
    4040                    4045                    4050

Leu Tyr Asp Tyr Val Asn Lys Tyr His Trp Glu His Thr Gly Leu
    4055                    4060                    4065

Thr Leu Arg Glu Val Ser Ser Lys Leu Arg Arg Asn Leu Gln Asn
    4070                    4075                    4080

Asn Ala Glu Trp Val Tyr Gln Gly Ala Ile Arg Gln Ile Asp Asp
    4085                    4090                    4095

Ile Asp Val Arg Phe Gln Lys Ala Ala Ser Gly Thr Thr Gly Thr
    4100                    4105                    4110

Tyr Gln Glu Trp Lys Asp Lys Ala Gln Asn Leu Tyr Gln Glu Leu
    4115                    4120                    4125

Leu Thr Gln Glu Gly Gln Ala Ser Phe Gln Gly Leu Lys Asp Asn
    4130                4135                4140

Val Phe Asp Gly Leu Val Arg Val Thr Gln Lys Phe His Met Lys
    4145                4150                4155

Val Lys His Leu Ile Asp Ser Leu Ile Asp Phe Leu Asn Phe Pro
    4160                4165                4170

Arg Phe Gln Phe Pro Gly Lys Pro Gly Ile Tyr Thr Arg Glu Glu
    4175                4180                4185

Leu Cys Thr Met Phe Ile Arg Glu Val Gly Thr Val Leu Ser Gln
    4190                4195                4200

Val Tyr Ser Lys Val His Asn Gly Ser Glu Ile Leu Phe Ser Tyr
    4205                4210                4215

Phe Gln Asp Leu Val Ile Thr Leu Pro Phe Glu Leu Arg Lys His
    4220                4225                4230

Lys Leu Ile Asp Val Ile Ser Met Tyr Arg Glu Leu Leu Lys Asp
    4235                4240                4245

Leu Ser Lys Glu Ala Gln Glu Val Phe Lys Ala Ile Gln Ser Leu
    4250                4255                4260

Lys Thr Thr Glu Val Leu Arg Asn Leu Gln Asp Leu Leu Gln Phe
    4265                4270                4275

Ile Phe Gln Leu Ile Glu Asp Asn Ile Lys Gln Leu Lys Glu Met
    4280                4285                4290

Lys Phe Thr Tyr Leu Ile Asn Tyr Ile Gln Asp Glu Ile Asn Thr
    4295                4300                4305

Ile Phe Asn Asp Tyr Ile Pro Tyr Val Phe Lys Leu Leu Lys Glu
    4310                4315                4320

Asn Leu Cys Leu Asn Leu His Lys Phe Asn Glu Phe Ile Gln Asn
    4325                4330                4335

Glu Leu Gln Glu Ala Ser Gln Glu Leu Gln Gln Ile His Gln Tyr
    4340                4345                4350

Ile Met Ala Leu Arg Glu Glu Tyr Phe Asp Pro Ser Ile Val Gly
    4355                4360                4365

Trp Thr Val Lys Tyr Tyr Glu Leu Glu Glu Lys Ile Val Ser Leu
    4370                4375                4380

Ile Lys Asn Leu Leu Val Ala Leu Lys Asp Phe His Ser Glu Tyr
    4385                4390                4395

Ile Val Ser Ala Ser Asn Phe Thr Ser Gln Leu Ser Ser Gln Val
    4400                4405                4410

Glu Gln Phe Leu His Arg Asn Ile Gln Glu Tyr Leu Ser Ile Leu
    4415                4420                4425

Thr Asp Pro Asp Gly Lys Gly Lys Glu Lys Ile Ala Glu Leu Ser
    4430                4435                4440

Ala Thr Ala Gln Glu Ile Ile Lys Ser Gln Ala Ile Ala Thr Lys
    4445                4450                4455

Lys Ile Ile Ser Asp Tyr His Gln Gln Phe Arg Tyr Lys Leu Gln
    4460                4465                4470

Asp Phe Ser Asp Gln Leu Ser Asp Tyr Tyr Glu Lys Phe Ile Ala
    4475                4480                4485

Glu Ser Lys Arg Leu Ile Asp Leu Ser Ile Gln Asn Tyr His Thr
    4490                4495                4500

Phe Leu Ile Tyr Ile Thr Glu Leu Leu Lys Lys Leu Gln Ser Thr
    4505                4510                4515

Thr Val Met Asn Pro Tyr Met Lys Leu Ala Pro Gly Glu Leu Thr
    4520                4525                4530

Ile Ile Leu
    4535

<210> 2
<211> 10
<212> PRT
<213> Homo sapiens

<400> 2

Val Pro Ser Cys Lys Leu Asp Phe Arg Glu
1               5                   10

## Description

### Technical Field

The present invention relates to a predicting factor for vascular aging and utilization thereof. In particular, the present invention relates to a predicting factor for vascular aging using glutathionylation of a specific thiol group of a human apolipoprotein B100 as an index and a detection method thereof.

### Background Art

In the metabolic syndrome which is expected to spread more and more in the future, diabetes mellitus (impaired glucose tolerance), hypertension, hyperlipidemia, obesity, and the like which are underlying diseases for the metabolic syndrome act commonly as a promoting factor for vascular aging. Vascular aging causes cardiac infarction and cerebral infarction through development of arterial sclerosis, arterial occlusion or the like. However, objective indices available for the vascular aging are those obtained by morphological measurements such as ultrasonic diagnostics of the carotid artery, and a simple measurement method using serum of a patient as the sample has not been established yet. That is, factors predicting onset of cerebral infarction, cardiac infarction, obstructive arteriosclerosis and the like which are critical and irreversible tissue damages subsequent to the metabolic syndrome have not been established to date.

Oxidative stress is thought to be one of the causes for many disorders including lifestyle-related diseases, and aging. Oxidative stress is also known to act as a promoting factor for vascular aging. As an oxidative stress marker, immunological determination of protein carbonylation and a TBAS method for determining lipoperoxidation are well known conventionally. However, these methods involve problems associated with specificity and reproducibility when serum is used as the sample. Meanwhile, in recent years, methods using a specific antibody against oxidative denaturation of lipid and protein fractions in LDL have been developed. The oxidative denaturation shows a process in which proteins and lipids undergo irreversible denaturation and degradation and is known to be associated with the advancement of atherosclerosis. However, the oxidative denaturation may not be considered in part as an index of earlier indication of vascular aging.

A thiol group of proteins is oxidatively modified in a nonenzymatic fashion by oxidative stress, which causes intramolecularly cross-linked protein and inactivation of enzyme proteins, thus proceeding to functional changes of proteins. A thiol group is sometimes oxidized to cause irreversible modification. S-glutathionylation (protein-SSG, glutathionylation) plays a role of protecting functions and structures of proteins from such an irreversible oxidative modification. In other words, hydrogen peroxide modifies a thiol group of a cysteine of a protein and promotes an irreversible denaturation process with sulfenic acid (-SOH) and further with sulfinic acid (-SO₂H) and sulfonic acid (-SO₃H). Modification of a thiol group with glutathione is a reversible change and is also regulated by concentrations of reduced glutathione (GSH) and oxidized glutathione (GSSG). This is referred to as "redox". Redox is an important mechanism that is associated with the function or activity of living organisms or cells and controls cellular differentiation and proliferation, and cellular death. In other words, the oxidative modification of a thiol group of a protein indicates an imbalance in the redox state of a living organism. Failure of redox results in apoptosis of tissue cells, and further, remodeling, and is considered to be deeply involved in the mechanism of vascular aging.

Methods for measuring a thiol group of a protein modified with glutathione have been reported so far.
1) An immunological method using an anti-glutathionylated bovine albumin antibody (non-patent reference 1). This method involves problems associated with specificity and measurement sensitivity, and is not suited for practical application.
2) A method using a biotinylated glutathione S-transferase (non-patent reference 2). This is an application of the fact that the glutathione S-transferase recognizes a glutathionylated thiol group through an enzyme reaction, but this method involves many problems associated with sensitivity and specificity.
[non-patent reference 1] Heille, OP et al., Eur. J. Neurosci., vol. 6, p. 793-804 (1994)
[non-patent reference 2] Cheng, G et al., Archiv. Biochem. Biophys., vol. 435, p. 42-49 (2005)

### Disclosure of the Invention

### Problems to be Solved by the Invention

An object of the present invention is to provide a biomarker useful for prediction of vascular aging and an examination method for a lesion resulting from vascular aging using the biomarker as an index.

### Means for Solving the Problems

The inventors of the present invention made intensive studies and examinations continuously in order to solve the above-mentioned problems, paid attention particularly to an apolipoprotein B100 localized in low density lipoprotein (LDL), identified the domain of a glutathionylated thiol group in advance, then produced a specific antibody against the site, and established a highly sensitive immunoassay to complete the present invention.

That is, the present invention provides the following:
[1] A predicting factor for vascular aging comprising an apolipoprotein B100 having a glutathionylated thiol group, or a part thereof which includes the glutathionylated region and consists of at least 10 amino acid residues,
[2] The predicting factor according to the above-mentioned [1], wherein the apolipoprotein B100 and a part thereof include at least an amino acid sequence represented by SEQ ID NO: 2, wherein the glutathionylated region is a thiol group of a cysteine in the amino acid sequence,
[3] A method for examining a lesion resulting from vascular aging comprising measurement of an apolipoprotein B100 having a glutathionylated thiol group using a biological sample,
[4] The method according to the above-mentioned [3], wherein the biological sample is blood,
[5] The method according to the above-mentioned [3] or [4], wherein the lesion resulting from vascular aging is accompanied by a disorder selected from the group consisting of diabetes mellitus, cerebrovascular disorder, cardiovascular disorder, obstructive arteriosclerosis, dementia, atherosclerosis, hypertension, obesity, and the like,
[6] An antibody specifically recognizing an apolipoprotein B100 having a glutathionylated thiol group, or a part thereof which includes the glutathionylated region, and consists of at least 10 amino acid residues,
[7] An antibody specifically recognizing glutathionylation of a thiol group of a cysteine in an amino acid sequence represented by SEQ ID NO: 2, and
[8] A diagnostic agent or a diagnostic kit for a lesion resulting from vascular aging comprising an antibody specifically recognizing an apolipoprotein B100 having a glutathionylated thiol group.

### Effects of the Invention

According to the present invention, since the correlation between an apolipoprotein B100 having a glutathionylated thiol group and a vascular lesion has been elucidated, an apolipoprotein B100 wherein a thiol group is modified or a part thereof can be used as a predicting factor for vascular aging. An examination method of the present invention using such a predicting factor as an index can make judgment briefly and with high accuracy that there is a lesion resulting from vascular aging or a risk for vascular aging, and is useful for diagnosis, follow-up, prognostic expectation, preclinical diagnosis, carrier diagnosis, or the like of a lesion resulting from vascular aging. Further, an antibody, and a diagnostic agent and a diagnostic kit comprising the antibody of the present invention can be used as a tool suited for the examination method of the present invention.

### Brief Description of the Drawings

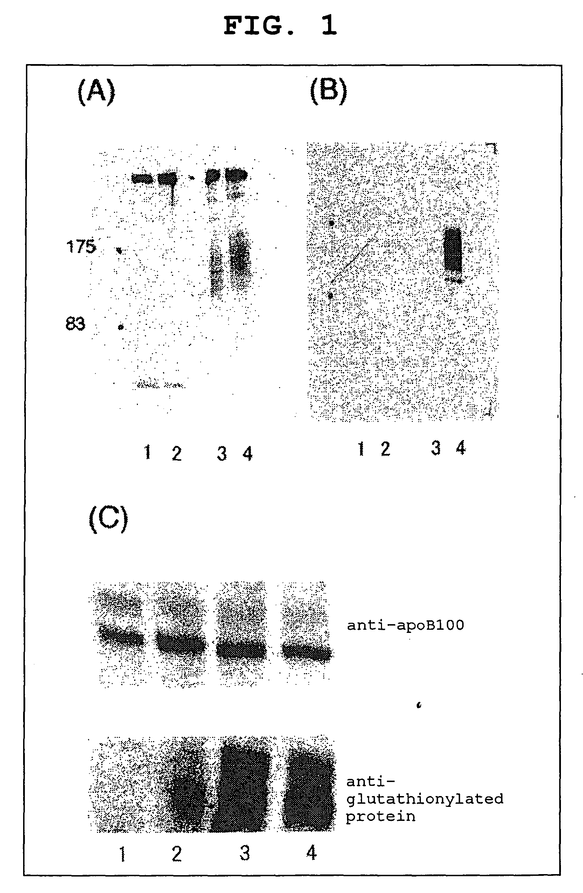

FIG. 1 is a drawing showing immunological measurement of a glutathionylated apolipoprotein B100 by SDS-slab gel electrophoresis. In (A), control serum from a healthy subject and serum from an ASO patient are used as samples, which are stained by an anti-apolipoprotein B100 antibody. Lane 1: Control serum + 5 mM DTT, Lane 2: Patient serum + 5 mM DTT, Lane 3: Control serum, Lane 4: Patient serum. In (B), control serum from a healthy subject and serum from an ASO patient are used as samples, which are stained by an anti-glutathionylated apolipoprotein B100 antibody. Lane 1: Control serum + 5 mM DTT, Lane 2: Patient serum + 5 mM DTT, Lane 3: Control serum, Lane 4: Patient serum. In (C), a glutathionylated apolipoprotein B100 produced in the control serum from a healthy subject is stained by an anti-apolipoprotein B100 antibody (upper line) and by an anti-glutathionylated apolipoprotein B100 antibody (lower line). Lane 1: 5% β-ME added, Lane 2: β-ME not added, Lane 3: 5 mM glutathione + 0.5 mM hydrogen peroxide added, Lane 4: 10 mM glutathione + 0.5 mM hydrogen peroxide added.
FIG. 2 is a drawing showing measurement of a glutathionylated apolipoprotein B100 in the serum from an ASO patient. (control (n=10) average 0.92±0.43; I (n=6) average 2.96±1.08; II (n=27) average 3.18±1.47; III, IV (n=18) average 3.97±1.73)
FIG. 3 is a drawing showing measurement of a glutathionylated apolipoprotein B100 in the serum from a diabetes patient. (control (n=10) average 1.07±0.18; no DM complication (n=12) average 0.91±0.41; DM, ASO(-) with other complication (n=52) average 2.24±1.90; DM, ASO(+) (n=26) average 2.71±1.49)
FIG. 4 is a drawing showing investigation of quantitative performance of an immune reaction using a purified glutathionylated apolipoprotein B100 as the antigen and using an anti-glutathionylated apolipoprotein B100 antibody. (A) is an electrophoresis photograph showing measurement results by the western blot method. (B) is a quantification graph showing the results of (A).
FIG. 5 is a drawing showing detection of a protein in the serum from a diabetes patient isolated by the immunoprecipitation method. (A) shows an anti-glutathionylated apolipoprotein B100 antibody and (B) shows an anti-apolipoprotein B100 antibody.
FIG. 6 is a drawing showing the thiol transferase activity in the serum from an ASO patient.

### Best Mode for Carrying Out the Invention

The present invention provides a novel predicting factor for vascular aging. The present invention targets a region that underwent reversible oxidative modification, rather than a modified protein that changes irreversibly by "oxidative stress" considered to particularly accelerate vascular aging. Living organisms have a high reduction power and thus protect themselves from oxidative stress. A sensor for such stress is, for example, a thiol group of a protein. Thus, the present invention takes note of the thiol group in blood protein and attempts to measure its modification.

An apolipoprotein B100 is a protein constituting a low-density lipoprotein (LDL), and in human beings, for example, it is a protein consisting of an amino acid sequence represented by SEQ ID NO: 1. Oxidative stress causes, in an apolipoprotein B100, irreversible modification to become a denatured LDL, which is taken into vascular endothelial cells or vascular macrophage by a scavenger receptor expressed on cellular surfaces of such cells, forms plaque in the blood vessel and thereby contributes to the development of an arteriosclerotic lesion. Further, oxidative stress causes diversified lesions including vascular aging. As will be described in examples shown later, a significant increase in apolipoprotein B100 having a glutathionylated thiol group was observed in the serum from a diabetes patient with a vascular lesion as compared to the protein in the serum from a diabetes patient without a vascular lesion. Therefore, an apolipoprotein B100 having a glutathionylated thiol group can be an excellent predicting factor for vascular aging.

The predicting factor of the present invention is characterized in that it comprises an apolipoprotein B100 having a glutathionylated thiol group, or a part thereof which includes the glutathionylated region, and consists of at least 10 amino acid residues. Specifically, a polypeptide containing an amino acid sequence is preferred wherein at least a thiol group in the cysteine in the amino acid sequence represented by SEQ ID NO: 2 (VPSCKLDFRE) is glutathionylated.

"Glutathionylation of a thiol group" as used herein denotes a thiol group of a cysteine of an apolipoprotein B100 in a -S-SG state reversible from an ordinary reduced form (-SH) due to oxidative stress or the like. This is different from a state that is further irreversibly modified by oxidative stress.

Vascular aging includes a physiological change of a blood vessel (artery) due to increased age, and a pathological change of a blood vessel induced by hypertension, hyperlipidemia, obesity, diabetes mellitus, smoking, cirrhosis hepatis, auto immune disease, or the like.

Further, the present invention relates to an examination method of a lesion resulting from vascular aging characterized in that an apolipoprotein B100 having a glutathionylated thiol group is detected using a biological sample. The method of the present invention allows not only detection of a possibility of a lesion resulting from vascular aging, but also the risk of a lesion resulting from vascular aging.

In lesions resulting from vascular aging, arteriosclerotic changes such as damage of vascular endothelial cells, thrombus formation, intimal thickening, vascular blockage and plaque formation are recognized.

As disorders accompanied by lesions resulting from vascular aging, diabetes mellitus, cerebrovascular disturbance, cardiovascular disturbance, obstructive arteriosclerosis, dementia, atherosclerosis, hypertension, obesity and the like are mentioned.

Although a subject to which the examination method of the present invention can be applied is preferably a human being, mammals other than a human being may be used. For such mammals, for example, mouse, rat, rabbit, canine, feline, equine, sheep, bovine, goat, swine, miniature pig, hairless pig, simian and the like are mentioned.

A test subject of the present invention is not particularly limited, and for human beings, those undergoing general medical check-up, those having a vascular aging risk, or those with lesions resulting from vascular aging and the like may be mentioned.

"Those having a vascular aging risk" preferably denotes human beings and the like with diabetes mellitus, hypertension, hyperlipidemia, obesity, or metabolic syndrome.

A biological sample applicable to the method of the present invention is a tissue or a body fluid in which an apolipoprotein B100 can exist, and is preferably a blood sample. As for the blood, any of whole blood, serum, and plasma may be used, and these may be obtained properly by treating blood taken from a subject by an ordinary method. Although not particularly limited, a blood sample is preferably serum. An apolipoprotein B100 having a glutathionylated thiol group may exist in the form of an "LDL containing an apolipoprotein B100 having a glutathionylated thiol group (glutathionylated LDL)", as well as an apolipoprotein B100 having a glutathionylated thiol group, while any of the forms can be measured by the method of the present invention.

In the present invention, detection of an apolipoprotein B100 having a glutathionylated thiol group in the blood sample isolated from a human being, is not particularly limited, and for example, methods such as an immunochemical method may be used. Among them, detection is preferably carried out by an immunochemical method that utilizes an antibody specifically recognizing an apolipoprotein B100 having a glutathionylated thiol group. Meanwhile, "detection of an apolipoprotein B100 having a glutathionylated thiol group" as used herein denotes not only qualitative detection of more than a certain concentration of an apolipoprotein B100 having a glutathionylated thiol group, but also quantitative measurement of the concentration of an apolipoprotein B100 having a glutathionylated thiol group.

There is no limitation for the immunochemical method used for measuring the concentration of an apolipoprotein B100 having a glutathionylated thiol group in a blood sample, and a conventionally known method such as dot-blot assay, western blot method, enzyme immunoassay (ELISA), latex agglutination assay, immuno-chromatography, radioimmunoassay (RIA), fluoroimmunoassay (FIA), and turbidimetry for measurement of turbidity accompanying the formation of an antigen-antibody complex are mentioned. Of them, it is preferable to determine the concentration of an apolipoprotein B100 having a glutathionylated thiol group from the difference of color development by the western blot method or dot-blot assay, while an antigen of a known concentration is used as the control.

When an apolipoprotein B100 having a glutathionylated thiol group is detected at more than a certain level in the blood sample, the subject from whom the blood sample is derived may be judged to have a lesion resulting from vascular aging or a risk for vascular aging. In this case, the higher the concentration of an apolipoprotein B100 having a glutathionylated thiol group in the blood sample, the higher the possibility or the risk that the subject has the lesion. Further, when an apolipoprotein B100 having a glutathionylated thiol group is detected at more than a certain level in the blood sample, it may be judged that the capability for reducing a glutathionylated apolipoprotein B100 in a living organism decreased in the subject from whom the blood sample is derived. In this case, the higher the concentration of an apolipoprotein B100 having a glutathionylated thiol group in a blood sample, the higher the possibility that the reducing power is decreased. On the other hand, when an apolipoprotein B100 having a glutathionylated thiol group is detected at less than a certain level in the blood sample, it may be judged that the subject from whom the blood sample is derived has a lower possibility or risk of having a lesion resulting from vascular aging, and that the capability of reducing a glutathionylated apolipoprotein B100 in living organisms is increased or maintained.

When an apolipoprotein B100 having a glutathionylated thiol group is detected in the blood sample, and when the amount of an apolipoprotein B100 having a glutathionylated thiol group in the blood sample is greater, it can be judged that a possibility or risk for having a lesion resulting from vascular aging is higher as compared to a case where the amount is less, and the possibility that the reducing power of a glutathionylated apolipoprotein B100 decreased in the living organism is higher.

Further, the present invention provides a diagnostic agent for a lesion resulting from vascular aging comprising a substance for detecting an apolipoprotein B100 having a glutathionylated thiol group.

As for the substance, although there is no limitation as long as the substance is capable of detecting an apolipoprotein B100 having a glutathionylated thiol group in the above-mentioned method, an antibody specifically recognizing an apolipoprotein B100 having a glutathionylated thiol group (hereafter, referred to as the "antibody of the present invention") is preferable.

The "antibody" in the present invention includes natural antibodies such as a polyclonal antibody and a monoclonal antibody (mAb), chimeric antibodies that can be produced using a genetic recombination technology, humanized antibodies and single-chain antibodies, human antibodies that can be produced using a human antibody producing transgenic animal or the like, antibody fragments produced by Fab expression library, and binding fragments thereof, but is not limited thereto.

The antibody of the present invention included in the diagnostic agent may be either a polyclonal antibody or a monoclonal antibody as long as it specifically binds to an apolipoprotein B100 wherein a thiol group derived from a mammal, preferably from a human being, is glutathionylated.

The binding fragment denotes a region of a part of the above-mentioned antibody, and specifically, for example, F(ab')₂, Fab', Fab, Fv (variable fragment of antibody), sFv, dsFv (disulphide stabilized Fv), dAb (single domain antibody) and the like are mentioned (Exp. Opin. Ther. Patents, Vol. 6, No. 5, p. 441-456, 1996).

The class of the antibody is not particularly limited and includes antibodies having any isotype such as IgG, IgM, IgA, IgD, IgE and the like. Preferably, IgG and IgM are mentioned, while IgG is more preferable when ease of purification and the like is taken into consideration.

A polyclonal antibody or a monoclonal antibody can be manufactured by an existing ordinary manufacturing method. That is, for example, an immunogen is given together with the Freund's Adjuvant as necessary to a mammal, e.g., mouse, rat, hamster, guinea pig, rabbit, feline, canine, swine, goat, equine or bovine, preferably mouse, rat, hamster, guinea pig, goat, equine or rabbit (in the case of polyclonal antibody) for immunization, and the same is given to mouse, rat or hamster (in the case of monoclonal antibody).

The immunogen is used cross-linked to a carrier, as necessary. For carriers, for example, BSA, KLH, and the like are mentioned. Further, a protein derived from animals to be immunized (e.g., a serum protein and the like) may be used as the carrier.

As for the immunogen used for production of an antibody of the present invention, an amino acid sequence represented by SEQ ID NO: 1 or a polypeptide having a partial sequence thereof is used. Preferably, the polypeptide comprises an amino acid sequence in which the thiol group of the cysteine in SEQ ID NO: 2 (VPSCKLDFRE) is glutathionylated or a partial sequence thereof. Specifically, the immunogen to be used for production of the antibody of the present invention is a peptide consisting of an amino acid sequence represented by SEQ ID NO: 2 (VPSCKLDFRE) or a partial sequence thereof. Although the length of the partial sequence is not limited as long as it possesses immunogenicity as an epitope, the length is preferably not less than 6 amino acids, more preferably not less than 8 amino acids, further preferably 10 amino acids.

For the sake of cross-linking the above-mentioned peptide to a carrier, one or a plurality of amino acids may be added. Although the number of amino acids to be added is not particularly limited, in consideration of the specificity of the antibody to be produced, the number of amino acids is preferably 1 to 10, more preferably 1 to 5, further preferably 1 to 2, most preferably 1.

Although the position of the amino acid to be added may be either an N-terminal or a C-terminal of the polypeptide, the amino acid is preferably added to an N-terminal.
Although the type of the amino acid to be added may be any of 20 types of amino acids known per se, preferably, at least one cysteine is included in the amino acid to be added. More preferably, the amino acid to be added consists of a cysteine.

Specifically, the polyclonal antibody can be produced as follows: that is, an immunogen is injected one to several times to a mouse, a rat, a hamster, a guinea pig, a goat, a horse or a rabbit, preferably a goat, a horse or a rabbit, more preferably a rabbit through subcutaneous, intramuscular, intravenous, foot pad, or intraperitoneal routes, and the mammal is sensitized. Normally, immunization is performed once to five times approximately every 1 to 14 days after the first immunization, and serum is obtained from the mammal that is sensitized about 1 to 5 days after the final immunization.

While the serum may be used as the polyclonal antibody, preferably, the antibody is isolated and/or purified by saturated ammonium sulfate, an euglobulin sedimentation method, a caproic acid method, a caprylic acid method, ion-exchange chromatography (DEAE or DE52, etc.), anti-immunoglobulin column or protein A/G column, or affinity column chromatography using a column or the like in which an immunogen is cross-linked.

A monoclonal antibody is produced by preparing a hybridoma (fused cell) from the antibody-producing cell obtained from the above-mentioned sensitized animal and a myeloma cell without auto-antibody producibility (myeloma cell), cloning the hybridoma, and selecting a clone that produces a monoclonal antibody exhibiting specific affinity to the immunogen used for immunization of the mammal.

Specifically, the monoclonal antibody can be produced as follows: that is, a mouse, a rat or a hamster (including transgenic animals created so as to produce an antibody derived from other animals such as a human antibody producing transgenic mouse) is sensitized by one to several times of injections of the immunogen through subcutaneous, intramuscular, intravenous, foot pad, or intraperitoneal routes or by grafting the same. Normally, immunization is performed once to four times approximately every 1 to 14 days after the first immunization, and antibody-producing cells are obtained from the mammal that is sensitized about 1 to 5 days after the final immunization.

Preparation of a hybridoma (fused cell) that secretes a monoclonal antibody can be performed according to the method proposed by Kohler and Millstein (Nature, Vol. 256, p. 495-497, 1975) and a modification method conforming to the method. In other words, antibody-producing cells contained in the spleen, lymph node, bone marrow or tonsilla, or the like obtained from the sensitized mammal as mentioned above, preferably antibody-producing cells contained in the spleen, and myeloma cells without auto-antibody producibility derived from preferably a mammal such as a mouse, a rat, a guinea pig, a hamster, a rabbit or a human being, more preferably from a mouse, a rat or a human being, are subject to cell fusion to prepare such a hybridoma.

For myeloma cells used for cell fusion, for example, myeloma P3/X63-AG8. 653 (653; ATCC No. CRL1580), P3/NSI/1-Ag4-1(NS-1), P3/X63-Ag8.U1(P3U1), SP2/0-Ag14(Sp2/0, Sp2), PAI, F0 or BW5147, derived from a mouse, myeloma 210RCY3-Ag. 2. 3. derived from a rat, or myeloma U-266AR1, GM1500-6TG-A1-2, UC729-6, CEM-AGR, D1R11 or CEM-T15, derived from a human being, may be used.

Screening of hybridoma clones producing a monoclonal antibody can be performed by culturing hybridoma in, for example, a microtiter plate, and measuring the reactivity of the culture supernatant of the well where proliferation is observed, against the immunogen used in the above-mentioned sensitization by enzyme immunoassay, for example, RIA or ELISA and the like.

Production of a monoclonal antibody from the hybridoma can be performed by culturing the hybridoma in vitro, or in vivo in a mouse, a rat, a guinea pig, a hamster or a rabbit or the like, preferably a mouse or a rat, more preferably in vivo in peritoneal effusion and the like of a mouse, and isolating the antibody from the culture supernatant or peritoneal effusion of the mammal thus obtained.

When culturing is performed in vitro, the hybridoma is proliferated, maintained and stored to meet with various conditions such as characteristics of the cell species to be cultured, the purpose of the test and research, and the culture method, and a known culture medium such as those used for production of a monoclonal antibody in the culture supernatant or any culture medium induced and prepared from a known basal medium can be used.

The monoclonal antibody is preferably isolated and/or purified similarly as the above-mentioned polyclonal antibody.

A chimeric antibody can be produced by reference to, for example, "Experimental Medicine (Extra edition), Vol. 6, No. 10, 1988", JP-B-H03-73280, a humanized antibody can be produced by reference to, for example, JP-T-H04-506458, JP-A-62-296890, and a human antibody can be produced by reference to, for example, Nature Genetics, Vol. 15, p. 146-156, 1997, Nature Genetics, Vol. 7, p. 13-21, 1994, JP-T-H04-504365, WO94/25585, "Nikkei Science, June, pp 40-50, 1995", Nature, Vol. 368, p. 856-859, 1994, JP-T-H06-500233, respectively.
F(ab')₂ and Fab' can be produced by treating immunoglobulin with pepsin and papain, respectively, which are proteolytic enzymes.

The antibody of the present invention produced by the above-mentioned method is able to recognize a human apolipoprotein B100 having a glutathionylated thiol group with extremely high sensitivity and specificity and is therefore very useful for detection and quantitative determination of an apolipoprotein B100 having a glutathionylated thiol group in a biological sample derived from a human.

The antibody of the present invention is included in the diagnostic agent in a free state, a labeled state, or a solid-phased state.

Use of the diagnostic agent of the present invention allows diagnosis of lesions resulting from vascular aging by the above-mentioned methods.

The diagnostic agent of the present invention can also be used as a diagnostic kit of lesions resulting from vascular aging which further comprises a reagent or the like used in the above-mentioned detection method. For the reagent or the like, specifically, a buffer solution for dilution of reagents and biological samples, a fluorescence dye, a reaction vessel, a positive control, a negative control, an instruction manual describing the inspection protocol and the like are mentioned. These elements may be mixed in advance as necessary. Use of the kit allows simple diagnosis of lesions resulting from vascular aging according to the present invention.

Contents of all publications including patent specifications cited herein are incorporated herein by reference in their entirety as if they had each been set forth in full.

Although the present invention will be described hereafter in detail referring to examples, the present invention is not limited to the following examples. In the examples shown hereafter an "apolipoprotein B100 having a glutathionylated thiol group" will be abbreviated as a "glutathionylated apolipoprotein B100".

### Examples

Apolipoprotein B100 protein C-terminal 10 amino acid residues (VPSCKLDFRE: SEQ ID NO.: 2) containing a reactive thiol group were synthesized (SIGMA). Glutathione (100 mM) was added to the synthesized peptide solution, which was then incubated at 37°C for 15 minutes in the presence of hydrogen peroxide (0.5 mM) for glutathionylation of the thiol group. The glutathionylation disappeared by reduction by treatment with a reducing agent and therefore, it was confirmed that the thiol group was in the state of becoming reversible -S-SG (glutathionylated). A specific antibody against a peptide having a glutathionylated thiol group was produced in a house rabbit (see Susanne Mohr et al., The Journal of Biological Chemistry, vol. 274(14), p. 9427-9430 (1999), Hjelle O. P. et al., Eur. J. Neurosci., vol. 6(5), p. 793-804 (1994) and Cheng G et al., Archives of Biochemistry and Biophysics, vol. 435, p. 42-49 (2005)).

### "Quantitative determination of glutathionylated apolipoprotein B100 in serum"

SDS-slab gel electrophoresis was performed in the absence of a reducing agent such as beta-mercaptoethanol (β-ME) or DTT (dithiothreitol) using human serum as a sample. It was stained with an anti-glutathionylated apolipoprotein B100 antibody and an anti-apolipoprotein B100 antibody using the western blot method and the degree of glutathionylation of the thiol group was determined.

### Results of experiments

### 1. Immunological measurement by SDS-slab gel electrophoresis (FIG. 1)

(A) Control serum from a healthy subject and serum from a patient with obstructive arteriosclerosis (ASO) were used as samples and stained with an anti-apolipoprotein B100 antibody (provided by SRL Ltd.). Bands corresponding to an apolipoprotein B100 were recognized only in the absence of DTT (lanes 3 and 4). The degree of staining was slight in the healthy control (lane 3) and was strong in the sample from an ASO patient (lane 4).
(B) The same samples were stained with an anti-glutathionylated apolipoprotein B100 antibody. They were stained with an anti-glutathionylated apolipoprotein B100 antibody in the absence of DTT. Although glutathionylation of the thiol group was observed with the fragmented apolipoprotein B100 fraction of an ASO patient serum, glutathionylation occurred also in a normal size apolipoprotein B100 that was not fragmented (lane 4). Meanwhile, it is known that an apolipoprotein B100 is fragmented during the course of oxidative denaturation due to oxidative stress and that lipid peroxides also increase (Cushing SD et al., Proc. Natl. Acad. Sci. USA, vol. 87, p. 5134-5138 (1990)).
(C) Preparation of glutathionylated apolipoprotein B100 using control serum from healthy subject
Hydrogen peroxide (0.5 mM) and glutathione (5 mM, 10 mM) were added to the control serum in the absence of β-ME, and reacted at 37°C for 24 hours. Then, presence of a glutathionylated apolipoprotein B100 that was not identified in the control was observed (lanes 3 and 4). The result thus obtained indicates that the thiol group of an apolipoprotein B100 is glutathionylated by oxidative stress.

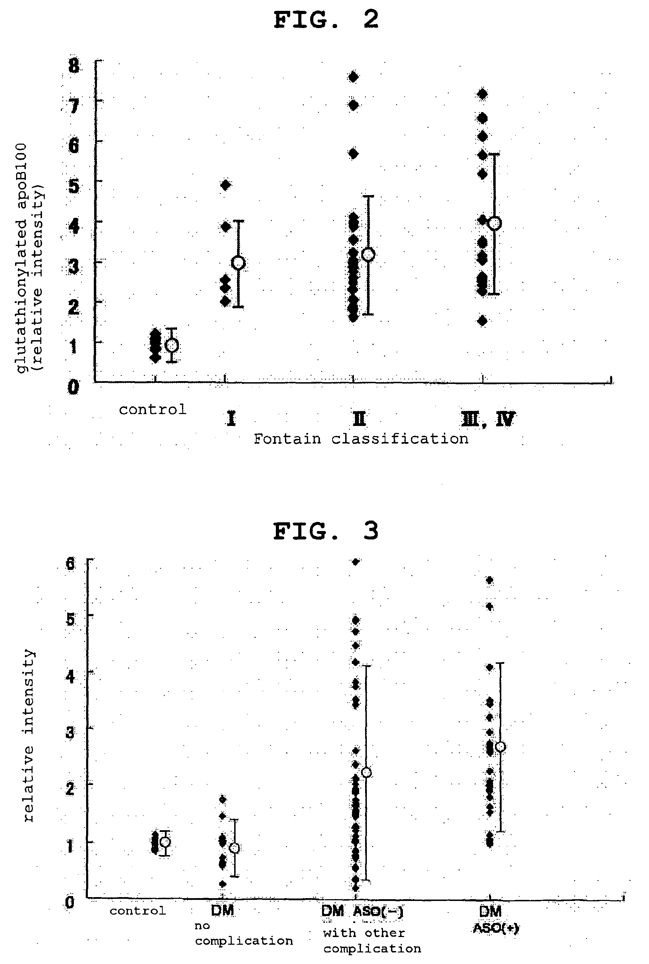

### 2. Measurement of glutathionylated apolipoprotein B100 in serum from ASO patient (FIG. 2)

Glutathionylated apolipoprotein B100 was measured using serum from an ASO patient as a sample and a significant increase in glutathionylated apolipoprotein B100 was observed with serum from an ASO patient as compared to the control serum from a healthy subject (p < 0.001). Pathological conditions of an ASO patient were investigated according to the Fontain classification, which revealed that there was no significant correlation, although an upward tendency of a glutathionylated apolipoprotein B100 was observed with the progress of the disease condition.
The results thus obtained suggest that determination of the concentration of a glutathionylated apolipoprotein B100 in the serum using the antibody of the present invention allows discrimination between healthy people and early-stage ASO patients.

### 3. Investigation of diabetic patients (FIG. 3)

Glutathionylated apolipoprotein B100 in diabetic patients (DM) was investigated with regard to presence or absence of a vascular lesion. A significantly correlative increase in glutathionylated apolipoprotein B100 was observed with the serum from diabetic patients with a vascular lesion or highly liable to have the same in the future as compared to the serum from diabetic patients without a vascular lesion or less liable to have the same in the future (Table 1).

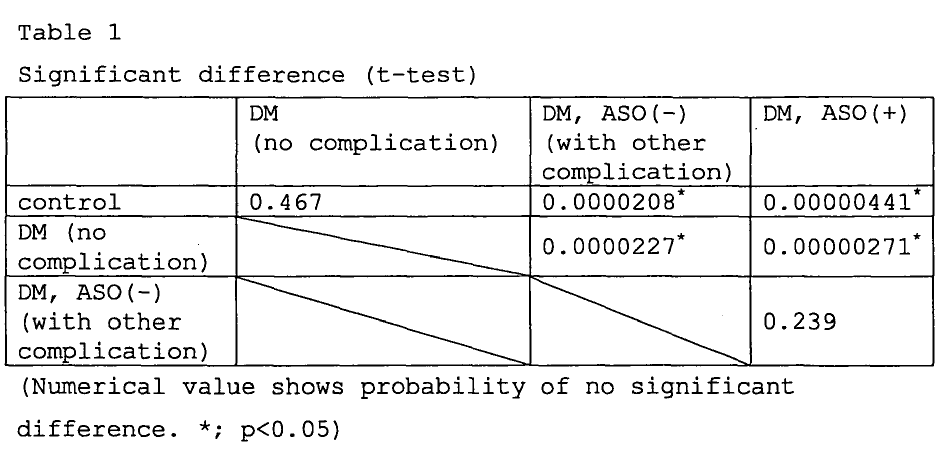

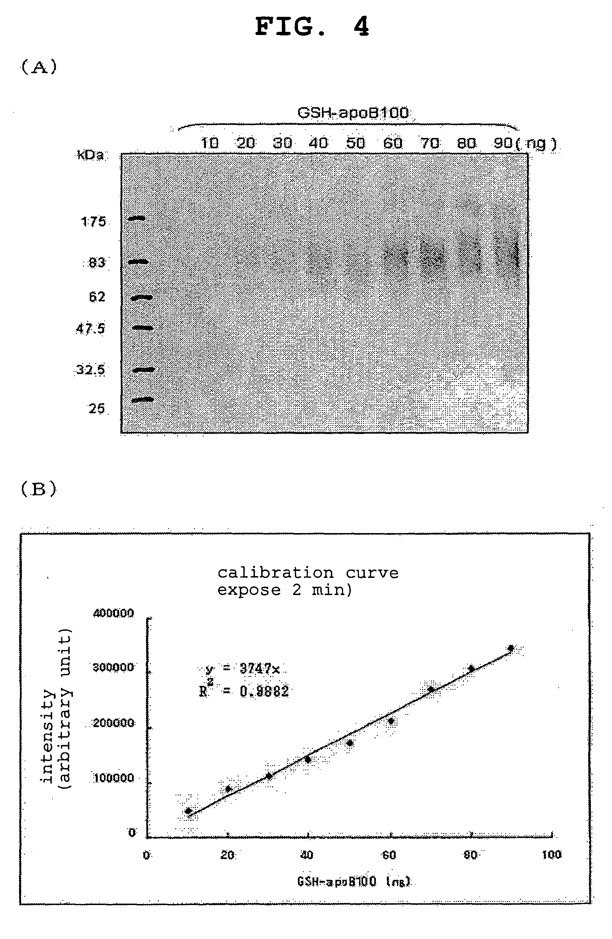

### 4. Quantitative performance of anti-glutathionylated apolipoprotein B100 antibody and antigen (purified glutathionylated apolipoprotein B100) in immune reaction (FIG. 4)

### (A) Quantitative determination of purified glutathionylated apolipoprotein B100 by western blot method using anti-glutathionylated apolipoprotein B100 antibody

A glutathionylated apolipoprotein B100 (GSH-apoB100) equivalent to 10 ng to 90 ng was subjected to electrophoresis and was assayed by the western blot method. An anti-glutathionylated apolipoprotein B100 antibody was diluted 5000-fold to be used as the antibody and was reacted at room temperature for 1 hour. Intensive bands were observed at locations corresponding to glutathionylated apolipoprotein B100 fractions.

### (B) Analysis of data of (A)

The results of (A) were quantified to obtain a calibration curve. As a result, a calibration curve showing antigen-level-dependent linearity was obtained.

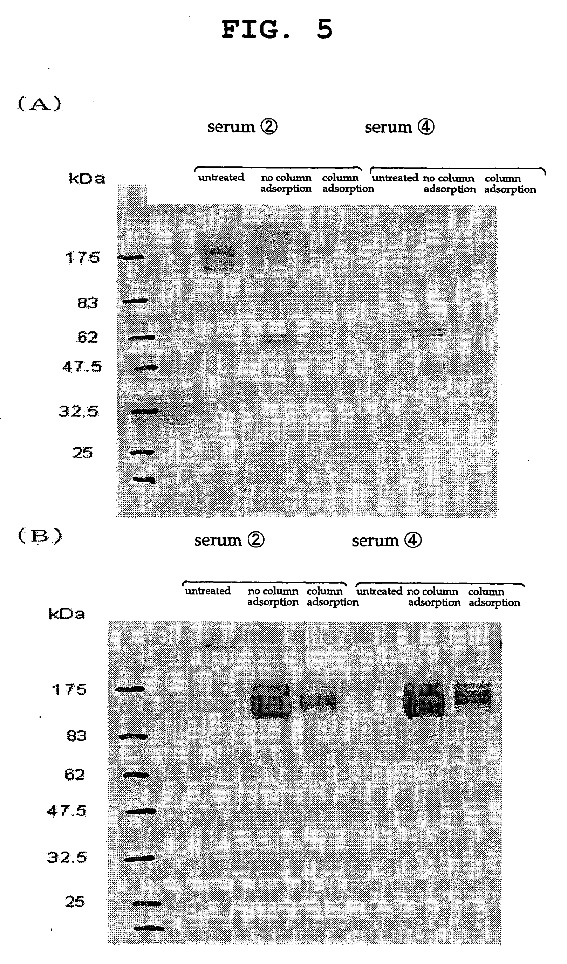

### 5. Extraction of protein that reacts with anti-apolipoprotein B100 antibody from patient serum using immunoprecipitation method and confirmation of effects by causing anti-glutathionylated apolipoprotein B100 antibody to act thereon (FIG. 5)

The serum from patients and an anti-apolipoprotein B100 antibody (SRL Ltd.) were caused to react and a protein that reacts with an anti-apolipoprotein B100 antibody was separated and extracted using an immunoprecipitation method using protein A sepharose. Following this, each of original serum (untreated), a protein not reacted with an anti-apolipoprotein B100 antibody (not adsorbed by column), and a protein bound to an anti-apolipoprotein B100 antibody (adsorbed by column) was subjected to electrophoresis, and an anti-glutathionylated apolipoprotein B100 antibody (FIG. 5 (A)) and an anti-apolipoprotein B100 antibody (FIG. 5 (B)) were caused to act. The protein immunoprecipitated by an anti-apolipoprotein B100 antibody was detected around 175 kDa for both anti-glutathionylated apolipoprotein B100 antibody and anti-apolipoprotein B100 antibody, which were approximately correspondent to each other. This finding revealed that a band detected by an anti-apolipoprotein B100 antibody is specifically detected by an anti-glutathionylated apolipoprotein B100 antibody.

From the results thus obtained, it has been found that the measurement method using the antibody of the present invention has excellent sensitivity and specificity as compared to the conventional methods. If a measurement method as a stress sensor is established, extensive clinical applications as a novel vascular aging marker would become possible.

### 6. Provision of predicting factor for vascular aging and of examination method of early-stage lesion resulting from vascular aging

The meaning of measurement of a glutathionylated apolipoprotein B100 as a risk factor for obstructive arteriosclerosis was investigated.
Using serum from 41 patients with obstructive arteriosclerosis and from 38 controls, a glutathionylated apolipoprotein B100 was measured and investigated by a Yates continuity-corrected x² test (Table 2).
It is apparent that a glutathionylated apolipoprotein B100 is an assessment factor having both extremely high sensitivity and specificity as compared to hs-CRP value that is already known to elevate in this disorder, and soluble LOX-1 (sLOX-1) value that is appreciated as an early diagnosis marker for vascular endothelial cell disorders such as cardiac infarction.

**Table 2**

| Sensitivity and specificity of sLOX-1, hs-CRP, and S-glutathionylated protein for ASO patients | | | |
|---|---|---|---|
| | sLOX-1 | hs-CRP | S-glutathionylated protein |
| Control (n=38) | | | |
| Positive, n | 21 | 13 | 4 |
| Specificity ASO (n=41) | 45 | 66 | 89 |
| Positive, n | 31 | 18 | 28 |
| χ² | 2.78 | 0.42 | 24.96 |
| *P* | 0.095 | 0.515 | <0.0001 |
| Sensitivity | 76 | 44 | 68 |

Cut-off levels are 100 pg/ml for sLOX-1, 4 µl/ml for hs-GRP, and 2.5 relative intensity for thiol-modification.
χ² was determined by a Yates continuity-corrected χ² test, and the probability value was obtained from comparison with non-ASO patients.

### 7. Meaning as index for decrease of blood redox ability which is the basis of vascular aging

Investigation was made as to what a glutathionylated apolipoprotein B100 reflects in a living organism.

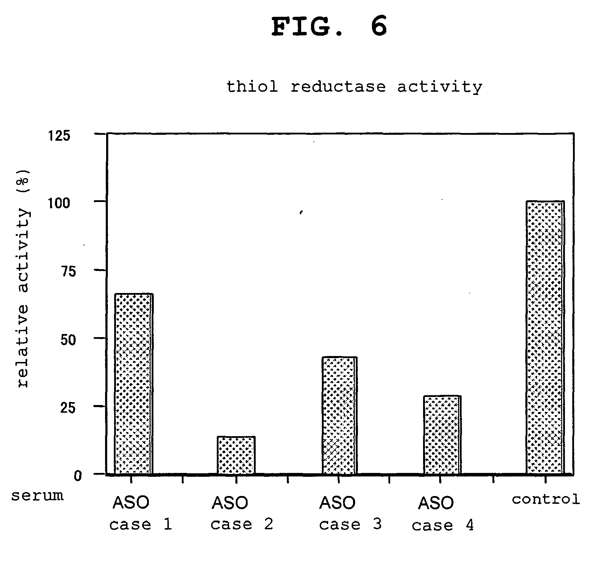

In the serum, vitamins which are antioxidative substances, glutathione, glutathione peroxidase, thioredoxin and glutaredoxin (GRX), which are redox proteins, and the like are present. Although these substances are considered to execute indirectly reduction of a glutathionylated serum protein, they do not directly reduce glutathionylated serum protein. The present inventors focused attention on GRX that has a direct reduction action on a cysteine group oxidatively modified in a glutathionylated protein, and have developed a method for measuring the activity of GRX dependent thiol transferase in the serum using low molecular weight protein tyrosine phosphatase (LMW-PTP) (Kanda, M, Kondo, T. et al., J. Biol. Chem., vol. 281(39), p. 28518-28528, 2006) which is a tyrosine phosphatase discovered by the present inventors to be a substrate of GRX. Specifically, LMW-PTP (100 µl/50 µg (2.8 nmols)) and ³⁵S-GSH (5 µl (2 pmoles, 50 nmols DTT)) were mixed and placed on ice for 5 minutes, then GSSG (28 nmoles) and H₂O₂ (100 nmols) (total amount 150 µl) were added thereto and the mixture was left standing at 4°C for 24 hours and filtrated by spin column to yield ³⁵S-GS-LM-PTP (PTP labeled). ³⁵S-GS-LM-PTP (5 µl) thus obtained and serum (0.5 µl) were mixed, 0.2 M NaH₂PO₄ (1 µl) (adjust pH to 7.0) and 7.5 mM GSH or PBS (-) (1 µl) were added thereto and left at 37°C for 30 minutes, a 2 × laemmli's solution (7.5 µl) was added to obtain a measurement mixture, and were subjected to the measurement of radioactivity released from a cysteine group of radiolabeled glutathionylated LMW-PTP. As a result, all patients with obstructive arteriosclerosis showed reduced activity of GRX dependent thiol transferase in the blood as compared to that of the control (FIG. 6). This suggests that the reduction power decreases in the patient serum, a protein SH group is in a state easily modified with oxidative stress, and that a power for reducing those oxidatively modified (glutathionylated) is low.

### 8. Prediction of vascular lesion progression unrecognizable by other measurement methods

### Assessment of glutathionylated apolipoprotein B100 as vascular lesion predicting factor

Although definite diagnosis of obstructive arteriosclerosis by angiography after progression of the lesion has been developed, prediction of an early stage of onset and incipient stage is not at all sufficient. An ABI inspection is objectively assessed at an early stage. In patients with obstructive arteriosclerosis, an extremely high positive correlation is observed between the ABI level and the glutathionylated apolipoprotein B100 level. Further, determination of the glutathionylated apolipoprotein B100 level of serum from patients with diabetes mellitus, which is one of the incipient disorders of this disease, revealed elevation of the glutathionylated apolipoprotein B100 level even with those who did not show any apparent symptom or change in the ABI level. They are considered to be in incipient stage and require careful follow-up in the future (data not shown). Meanwhile, though in one case, the glutathionylated apolipoprotein B100 level of a diabetic patient who complained of intermittent pain in the lower legs was measured and found to be normal. In this case, the patient had a complication of marked hypertension, obesity, and hyperlipidemia, and was strongly suspected of suffering obstructive arteriosclerosis. However, since the ABI level was also normal, the possibility of other disorder was strongly suggested. From this incident, it is expected that a more accurate diagnosis will be enabled by determination of the glutathionylated apolipoprotein B100 level in addition to the sole determination of ABI.

### Industrial Applicability

It is considered that a method of determining, as a stress sensor, a serum apolipoprotein B100 protein having a glutathionylated thiol group can be extensively used in clinical applications as a novel vascular aging marker. Metabolic syndrome patients constantly increasing in number year by year may encounter serious lifestyle-related diseases, which include cerebral infarction, cardiac infarction, obstructive arteriosclerosis and the like due to vascular aging. In addition, a vascular lesion underlies a complication of diabetes mellitus such as blindness and impairment of renal function. Factors for predicting onset of these disorders have not been established so far. It is expected that the method of determining a vascular aging marker established by the present invention would make great contribution to determination of prognosis of metabolic syndrome patients as well as diabetic patients. Further, development of a screening system for anti-arteriosclerotic agents using this probe is also possible. It is also expected that the determination method of the present invention would be applicable to objective diagnosis and judgment of therapeutic effects of dementia which is supposed to become a serious social problem in the future.
This application is based on a patent application No. 2005-344630 filed in Japan (filing date: November 29, 2005), the contents of which are incorporated in full herein by this reference.

## Claims

1. A predicting factor for vascular aging comprising an apolipoprotein B100 having a glutathionylated thiol group, or a part thereof which includes the glutathionylated region and consists of at least 10 amino acid residues.

2. The predicting factor of claim 1, wherein the apolipoprotein B100 and a part thereof include at least an amino acid sequence represented by SEQ ID NO: 2, wherein the glutathionylated region is a thiol group of a cysteine in the amino acid sequence.

3. A method for examining a lesion resulting from vascular aging comprising measurement of an apolipoprotein B100 having a glutathionylated thiol group using a biological sample.

4. The method of claim 3, wherein the biological sample is blood.

5. The method of claim 3 or 4, wherein the lesion resulting from vascular aging is accompanied by a disorder selected from the group consisting of diabetes mellitus, cerebrovascular disorder, cardiovascular disorder, obstructive arteriosclerosis, dementia, atherosclerosis, hypertension, obesity, and the like.

6. An antibody specifically recognizing an apolipoprotein B100 having a glutathionylated thiol group, or a part thereof which includes the glutathionylated region, and consists of at least 10 amino acid residues.

7. An antibody specifically recognizing glutathionylation of a thiol group of a cysteine in an amino acid sequence represented by SEQ ID NO: 2.

8. A diagnostic agent or a diagnostic kit for a lesion resulting from vascular aging comprising an antibody specifically recognizing an apolipoprotein B100 having a glutathionylated thiol group.
